# EUROPEAN PATENT APPLICATION

(11) **EP 3 797 725 A1**
(43) Date of publication of application: **31.03.2021**
(21) Application number: 19390001.6
(22) Date of filing: 24.09.2019
(51) Int. Cl.: A61B 34/30, A61B 5/055, A61N 7/02, A61B 90/50

(54) **HIGH INTENSITY FOCUSED ULTRASOUND ROBOTIC SYSTEM OPERATING IN MAGNETIC RESONANCE ENVIRONMENT**

(71) Applicant: Medsonic Limited, 4041 Limassol (CY)
(72) Inventor: Giannakou, Marinos, 3025 Limassol (CY)
(74) Representative: Rousounidou, Vasiliki A.

(57) **Abstract**

The current invention relates to an MRI guided robotic system for focused ultrasound dedicated to kill tumors in the abdominal area (kidney, liver, pancreas), and in prostate. The robotic system employs materials that can function inside the MRI environment. Such materials such are ultrasonic motors, brass screws and ABS plastic. The robotic system can be attached to MRI table using 8 brass screws. The robotic system includes 4 PC-controlled axes for the abdominal area option. The prostate option includes 6 PC-controlled stages and one additional manual stage. The robotic system accesses the prostate via endorectal placement of the transducer probe. Access of ultrasound to the abdominal area is achieved from top to bottom. Access to the brain is also possible using a lateral approach. This is the first system that provides access to so many targets (brain, liver, kidney, pancreas, bone, thyroid, breast,) that are accessible by ultrasound. The motion of the robotic system is very accurate due to the use of MRI compatible encoders. Because the robotic system is fixed on the table of the MRI scanner, this system can be used in all the commercial MRI systems making it a universal robotic system. Another major advantage of this robotic system is that it is lightweight and therefore it can be transported as a portable.

## Description

### TECHNICAL FIELD

The present invention relates to a magnetic resonance imaging (MRI) compatible robotic system for navigating high intensity focused ultrasound (HIFU) transducer. The proposed robotic system can be used for treating brain, breast, bone, abdominal, and fibroid cancer.

### BACKGROUND ART

Prostate cancer (PC) is one of the most frequently diagnosed cancers in the male population in the world [1]. According to the American Cancer Society, PC represents 25 % of newly diagnosed cancers every year [2]. In Europe, the mortality rate for PC was 21.1 % in 2008 [2]. Tumor stage, grading of the tumor (Gleason score) and serum prostate-specific antigen (PSA) levels are the most important prognostic factors for PC [3].

Liver cancer is one of the most common cancer malignancies worldwide. Hepatocellular carcinoma (HCC) is the prevailing form of primary liver tumour accounting for 80-90% of the cases [4]. In 2018, a total of 841,080 new cases were reported worldwide, accounting for 4,7 % of all cancer incidences, thus making it the sixth most occurring malignancy form worldwide. The mortality cases were at 8.2 %, making it the fourth leading cause of cancer mortality globally [5]. The epidemiologic studies show a greater occurrence in middle aged men, predominantly in East-Asian and Sub-Saharan countries [6].

Renal cell carcinoma (RCC) is the predominant form of primary kidney malignancies, accounting for 90 % of the cases [7]. In 2018, a total of 403,262 new incidents were reported globally, making it the 14th most common form of cancer and the 16th cause of cancer mortality [8]. The incidences vary geographically, with percentages being higher in Europe and North America, and affecting almost double males than females [9].

Pancreatic adenocarcinoma is the main type of pancreatic cancer, accounting for 85 % of the incidents [10]. In 2018, an overall of 458,918 new cases were announced, making it the 12th occurring form of cancer malignancy [11]. However, it is the 7th leading cause of cancer mortality due to late diagnoses of the disease, usually at an advanced stage. The pancreatic malignancies usually appear in men after their 6th decade of life, with the majority of cases appearing in European and Eastern Asian countries [12].

Treatment options for PC include the following: radical prostatectomy (RP), external beam radiation therapy (EBRT), active surveillance, and interstitial prostate brachytherapy [13]. The European Association of Urology (EAU) guidelines recommend RP for intermediate and high-risk populations [14]. Active surveillance is recommended for the low risk population [14]. However surgical techniques have numerous complications that appear in about one in four cases, which include impotence, incontinence, urinary tract infections and often require lengthy hospitalization.

A recently published randomized trial that compared retropubic RP to "watchful waiting" showed that RP reduced PC mortality and the risk of metastases [15]. This wide spectrum of treatment option for PC arises from an incomplete comprehension of the PC biology [16]. Several studies demonstrated that low risk prostatic tumors are correlated with a better prognosis in terms of biochemical free survival and metastatic-free disease [14]-[16].

Standard treatment for PC has long been "wholegland" therapy including RP (complete surgical removal of the prostate) or radiation therapy of the entire prostate (either with external beam or brachytherapy). However, widespread PSA-based PC screening has led to a profound stage migration with a large proportion of men being diagnosed with a low-stage, low-grade cancer that has minimal risk of progression. Several men are diagnosed with a PC not destined to become clinically evident during their natural lifespan. There is growing evidence and clinical judgment that patients with low-risk disease may benefit significantly from a minimally invasive focal therapy and avoid complications associated with prostatectomy and other "whole gland" treatments. It is becoming clear that a number of patients with limited prostate cancer could be offered focal treatment in which only the focus of the tumour is targeted: between the two extremes of active surveillance and radical treatment, there must be a middle way.

Different energy sources are proposed to achieve whole gland, subtotal or focus ablation with the guidance of imaging modalities, such as ultrasound (US) or MRI. Brachytherapy and radiation external beam therapy are the most commonly used as minimally invasive techniques, not only for the therapy of localized PC, but also for the palliation of high-grade tumors [13]-[15]. Laser and cryoablation have also been used for focal therapy with promising results [13]-[15]. Despite the multifocal nature of prostate cancer, many affected men have a single substantial lesion also known as the index lesion [17].

In the 1990's focused ultrasound surgery (FUS) has been utilized for the treatment of PC [14],[18]. FUS is a non-invasive therapy that has been used so far for localized PC or salvage therapy. It is a technique that uses focused ultrasound waves to thermally ablate a portion of tissue situated at the focal point. Focused ultrasound can be focused on a targeted point to cause a rise in temperature between 60-80 °C. This can result in thermal tissue coagulation necrosis. Each sonication heats only a small focal target, so multiple sonications, must be used to ablate an entire target area [19]. Due to the propagation mechanism of ultrasound, this technique should be performed with caution near nerves, bone, and rectum.

Recently, MRgFUS has been introduced due to a better ability to plan and monitor treatments in real-time [20]. This technique is approved by the Federal and Drugs Administration (FDA) for fibroid ablation and shows great potential in bone metastasis pain palliation. Promising results for treatment of liver, breast and brain tumor and prostate, were also obtained [20].

The first FUS prototype was built in 1940 and the general technology existed in an experimental setting for over 50 years. However, in the 90's has this technology been employed for approved clinical applications. Two FUS devices are currently available for prostate ablation: Ablatherm (EDAP TMS SA, Vaulx-en-Velin, France) [21]-[22] and Sonablate (Focus Surgery Inc., Indianapolis, IN, USA, now called SONACARE) [3]. The Ablatherm has both the imaging (7.5 MHz) and therapeutic (3 MHz) transducers included in a unique endorectal probe focused at 40 mm. The Sonablate uses a single transducer (4 MHz) for both imaging and treatment. Several probes are available with many focal lengths (from 25 to 45 mm) [3],[21]. The prostate is visualized using real-time diagnostic images generated by the probe using lower, nondestructive acoustic energies (0.1-100 mW/cm2). Once the target areas are identified, the prostate tissue is ablated with high energies (1300-2200 W/cm2) focused in a small (1-3 mm wide by 5-26 mm long) focal plane. Each pulse heats the tissue to 70 to 90 oC over a 3-5 s period. The ultrasound frequency used in the existing systems is optimum for therapy, but is not very effective for imaging (bad spatial resolution). Both devices operate under ultrasound guidance.

Most patients treated with FUS presented with localized cancer [16]. Usually FUS is used as a stand-alone procedure with a 5-year disease-free survival rate (biochemical) of 77 % for Ablatherm [23]-[25] and 45-84 % for Sonablate [3].

FUS treatment could be complicated by adverse events involving the bladder function (2 %-58 %) [3]. Other complications included rectal burn (0-15 %) and rectourethral fistula (0-3 %). The studies performed using an Ablatherm device reported higher complication rates with respect to the Sonablate studies.

One of the first experiences in human prostate MRg-FUS was presented by Napoli et al. [26] where patients with localized PC underwent to MRgFUS with ExAblate system before RP. The system combines a 2.3 MHz, 1000 element/channel phased array transducer, and a robotic transducer positioning system. In subsequent studies by Cheng et al. [27] eighteen patients with low risk PC were treated with MRgFUS. Initial reports suggest that MRgFUS treatment is associated with low morbidity, including preservation of continence and erectile function.

Another approach for MRgFUS option is to use a transurethral probe. With this approach wider variety of applicators are used (planar, curvilinear and tubular). A planar applicator [28]-[29] can be used with faster penetration and large volume of treatment. The advantage of curvilinear applicators is greater control over selective heating. The tubular applicator [30]-[31] allows electronic control of an angular heating pattern. Both the planar and curvilinear applicator needed about 10-30 mins to ablate larger lesions in the prostate because of a narrow acoustic beam width (4 mm) [29]. The tubular applicator does not need mechanical rotation, unlike the others, so it may reduce potential motion artifacts [30]-[31].

Hepatectomy is the gold standard for HCC therapy. However, it can only be performed on 10-37 % of the patients. It has a 30 % probability of 5-year survival rate with a high percentage (70-85 %) of tumor recurrence. Liver transplantation was performed by Starlz et al. in 1963 [32]. The transplantation is better than hepatectomy in tumor recurrence percentages, but it has serious complications including tumor metastases and suppression of the immune system. These, along with the long waiting lists and minimal number of donors have made surgical resection the therapy of choice [33]. With the number of cancer cases increasing, there is a need for alternative minimally invasive treatments for therapeutic or palliative purposes.

Percutaneous ethanol injection (PEI) exists as a therapy since 1983 [34]. It is effective for small hepatocellular tumours. Nonetheless, it requires a number of sessions due to a high percentage of blood diffusion [35]. Radiofrequency ablation can be performed percutaneously or intraoperatively on tumours slightly larger than PEI. Studies have shown a 40-50 % of 5-year survival and 25-45 % of tumor recurrence [36]. Cryosurgery is another form of ablative therapy, slightly more advantageous than PEI and RFA, since it can be used near large tumours. However, it may induce symptoms, including but not limited to thrombosis, tumour haemorrhage and pleural effusion [37].

With the constantly increasing number of patients, the development of a less-invasive, more efficient and with minor post-treatment complications therapy had to be introduced. HIFU started developing as a technique since the 40's. In 1987, Frizzel et al. [38], were the first to study the required intensity to cause irreversible tissue damage on the liver. Since then, further animal studies were performed to assess the efficacy and safety of the therapy.

Seket et al. [39], analyzed the size and reproducibility of lesions on 5 pigs. Histological examination showed complete necrosis at the lesion centre, with large adjacent vessels maintaining their functionality. Chen et al. [40], used HIFU on 29 rats to examine the histological changes. The treated regions showed necrosis with signs of tumour cell survival in the safety margin area.

After the development of MRI in the 90's, a method was developed that could monitor the temperature change during the ablation, thus increasing its control. Hynynen was the first to use MR- guidance in 1993 [41]. Further animal studies were performed to assess the efficacy. Kopelman et al. [42], performed MRI guided HIFU lesions on pigs. The lesion areas showed complete necrosis. Further studies on pigs showed complete correlation between lesion size and calculation of thermal dose [43]. Jiang et al. [44], examined the effect of changes on the portal vein under MRgFUS on 50 goats. The results showed complete necrosis of the lesions, with no major changes of the portal vein and liver vessels.

Following efficacy and safety, clinical trials were performed using both ultrasound and MRI guidance. Chuang-Xing et al. [45], used a US guided equipment to examine the clinical effects of HIFU. Their study was performed on 100 patients with the majority of them showing necrosis, relief of symptoms and an increase of liver functioning enzymes. Another study using a US guidance system was performed on 55 patients with the majority of them not being suitable for surgery. Necrosis was achieved, with most of the patients showing reduction in tumor size 6 months after the HIFU ablation [46]. HIFU performed on 14 patients examined lesions acquired by HIFU ablations and compared with corresponding RFA. The rate of reduction in size was found to be the same, but with HIFU showing a decrease percentage of tumor recurrence [47]. Further follow-up studies performed, reported patients showing improvements on abdominal pain, fatigue and loss of appetite [48], with complete necrosis and minimal damage to adjacent blood vessels [49]. Zhang et al. [50], used Diffusion Weighted MR Imaging to examine the effects of necrosis rather than histopathology. Again, they found that the majority of patients showed signs of necrosis with a small percentage showing tumor recurrence. Some studies have performed partial rib resection, prior HIFU treatment, to provide a better acoustic window [51] for the complete ablation of the tumour. The only clinical trial involving MR guided HIFU was performed in 2006 on a single patient [41], concluding the future possibility of the combination, but with the need of technical advancement to account for organ movement and rib reflection [52].

Radical nephrectomy is the generally accepted treatment technique. However, it has a 40 % chance of tumor recurrence [53]. Open partial nephrectomy can be performed in renal cell carcinomas (RCCs) smaller than 4 cm. It has the same survival rates with radical nephrectomy, with the advantage of maintaining the renal function [54]. A technique, not widely used, with similar results to nephrectomy is simple enucleation [55]. Radiofrequency ablation and cryosurgery are also used, having the same complications and tumour recurrence as for treating hepatocellular carcinomas. All current techniques require puncture of renal tumour, with bleeding being a serious complication.

Several animal studies have been performed to account for the efficacy and safety of HIFU ablations for renal masses. Adams et al. [56] used HIFU on surgically exposed kidney tumours of rabbits. They concluded that their technology can be used to cause tissue necrosis, however, complete tumour ablation was limited by the poor imaging ultrasound system. Paterson et al. [57], laparoscopically used a HIFU system with ultrasound guidance for ablation on 13 mini-pigs. All treated kidneys were histologically examined and showed signs of necrosis with characteristic lesion size with no change in renal function. Watkin et al. [58], examined the possibility of a non-invasive ablation on kidney tumours in pigs. Distinct lesions appeared on 67 % of the animals. The percentage variation of tissue necrosis was due to attenuation of the ultrasound beam by the bones.

Hacker et al. [59], were the first to assess for non-invasive ablation with both animal and clinical trials. They used an ultrasound guided system for HIFU ablation on 12 dogs and 19 patients. Coagulation necrosis appeared in all subjects with no adverse side effects. Skin burns appeared on two patients and lesion formation on the small intestine of one dog due to incorrect coupling and unfocused beam. Vallancien et al. [60], were the first to use HIFU on renal masses of patients. The patients showed signs of necrosis, with the majority of them having severe skin burns. Wu et al. [61], performed HIFU on 12 patients, with all of them showing signs of necrosis and alleviation of pain, with no side effects.

Klingler et al., used an ultrasound guided HIFU system for a laparoscopic ablation on 10 patients prior to undergoing nephrectomy, with 80 % of the patients showing complete necrosis of the tumour tissue [62]. Marberger et al. [63], used two different systems to assess for their efficacy of non-invasive ablation. They used the Storz extracorporeal HIFU device on 16 patients. Necrosis appeared on all of the patients, with two having their tumour completely coagulated. All of the patients had a normal kidney surface, with none of them showing any adverse side effects. The 'Chongqing' extracorporeal system was used on 4 patients with renal tumours. All showed signs of necrosis with no skin burns despite the use of high intensities. Iling et al. [64], used an extracorporeal ultrasound guided system on 8 patients with renal masses. The patients showed signs of ablation, with a minority of them having a mild fever and formation of blister around the target area. No clinical trials have been found to use MRI guided HIFU. Saeed et al. [65], used MR guidance for HIFU ablation on kidney tumours of 6 pigs. All pigs had distinct lesions with signs of necrosis, with no serious damage to the surrounding tissue. MR guidance was useful for real-time temperature mapping, and diffusion imaging of the lesions.

Pancreatectomy remains the only treatment for complete therapy of pancreatic cancer. However, due to late diagnosis of the pancreatic cancer, surgery cannot be performed on all patients. Despite, the many years of advances, it still has high rates of mortality [66]. Due to the advanced stage of disease during diagnosis, chemotherapy and radiotherapy can be used only for palliative purposes and for increasing the patient's suitability for surgery [67]. The use of cryosurgery and radiofrequency ablation has the same limitations and side effects as when treating liver carcinoma [68].

Xie et al. [69], examined the possibility of using HIFU for pancreas on 12 pigs. The pigs did not have any tumour. Necrotic lesions appeared on the target margins, concluding the effective use of HIFU on pancreas. Mao et al. [70], used HIFU on 54 cats to examine for normality of liver functions. Cats were divided into groups, with some of them having up to 50% of their pancreas sonified. Necrosis was observed, with all cats having normal pancreatic functions after the treatment. Liu et al. [71], performed combination of HIFU and radiotherapy on 12 pigs to assess for the efficacy of the combination. They concluded that the combination of both treatments had a longer percentage of damage, however, with the pigs of that group showing signs of anorexia and skin burns. Jiang et al. [72], performed US guided HIFU on 18 mice having pancreatic tumours. After the insonification, the volume of the tumours was reduced by 100 %.

Xie et al. [73], performed a US guided HIFU for pancreatic cancer of 16 patients. Due to the advanced stage of the patients, none of the tumours were completely ablated. Only in 4 patients the tumour size was decreased. Nevertheless, all patients showed improvement in their appetite and pain. Atsushi et al. [74], performed HIFU on 30 patients being in advanced stage. Again, the patients only showed relief of symptoms, with no adverse side effects. Ge et al. [75], used HIFU on 20 patients to find the optimal tumour depth for ablation. It was concluded that tumours being at a depth smaller than 7 cm, had a larger reduction rate of the tumour, accounting for optimal ablation. Li et al. [76], performed ablations on 25 patients not suitable for surgery. Eight of them showed signs of necrosis, with the entirety of the patients showing pain relief post-treatment. Wu et al. [77], performed US guided HIFU on 8 patients in advanced stage, most of them having liver metastasis. All of the patients showed tumour reduction and their pain had stopped. Two of the patients also received HIFU on their liver metastasis, with both of them still alive 1-year post-treatment. In a study by Xiong et al. [78] 89 patients received HIFU ablations. Complete necrosis was not achieved in any of them, since the majority of the patients had a stage 4 cancer. Despite that, pain was alleviated in 80 % of them, with some having minor skin burns and fat sclerosis. Lee et al. [79], performed a study on 12 patients to examine the effects of using combined HIFU with chemotherapy. HIFU ablations were performed in 9 patients, with the rest of them acquiring both treatments. It was concluded that the combined treatment increased the patients' survival time.

### TECHNICAL PROBLEM TO BE SOLVED

Cancer is a major disease resulting to death, Therefore, there is a need for alternative therapies. What is proposed is a non-invasive system that uses HIFU to kill tumors under MRI guidance. With this system the patient can be placed in supine position.

### DISCLOSURE OF THE INVENTION

The current invention relates to an MRI guided robotic system for focused ultrasound dedicated to kill tumors in the abdominal area (kidney, liver, pancreas), and in prostate.

The robotic system employs materials that can function inside the MRI environment. Such materials such are ultrasonic motors, brass screws and ABS plastic. The robotic system can be attached to MRI table using 8 brass screws. The robotic system includes 4 PC-controlled axes for the abdominal area option. The prostate option includes 6 PC-controlled stages and one additional manual stage. The MRI compatible ultrasonic motors minimally affect the signal of the MRI scanner because are position quite far from the MRI coil.

The robotic system accesses the prostate via endorectal placement of the transducer probe. Access of ultrasound to the abdominal area is achieved from top to bottom. Access to the brain is also possible using a lateral approach. This is the first system that provides access to so many targets (brain, liver, kidney, pancreas, thyroid, breast, ) that are accessible by ultrasound. The motion of the robotic system is very accurate due to the use of MRI compatible encoders.

Because the robotic system is fixed on the table of the MRI scanner, this system can be used in all the commercial MRI systems making it a universal robotic system. The current devices are accommodated in the MRI table, thus the device volume has to me modified based on the MRI type. The system has high standards of safety, accuracy and precision.

Another major advantage of this robotic system is that it is lightweight and therefore it can be transported as a portable.

### BRIEF DESCRIPTION OF THE DRAWINGS

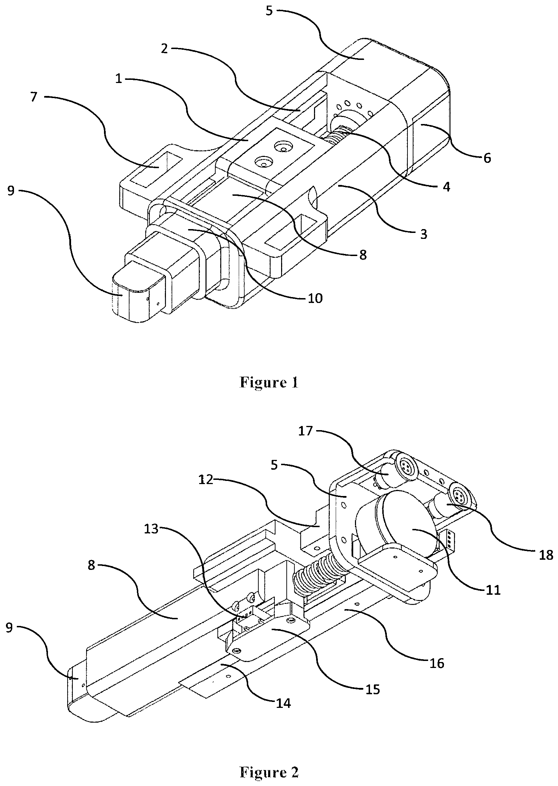

Fig.1 Z-stage of the robotic system that carries the Y-stage.
Fig.2 Z-stage mechanism and electronic components for motion accuracy and actuation of the robotic system.

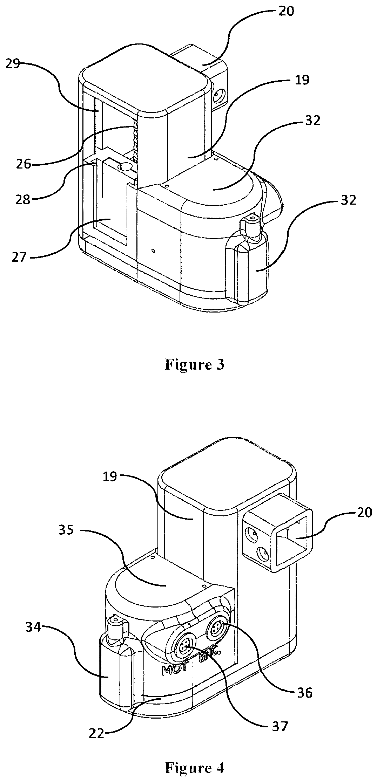

Fig.3 Y-stage of the robotic system that carries the X-stage.
Fig.4 Y-stage of the robotic system with the motor and encoder connectors.

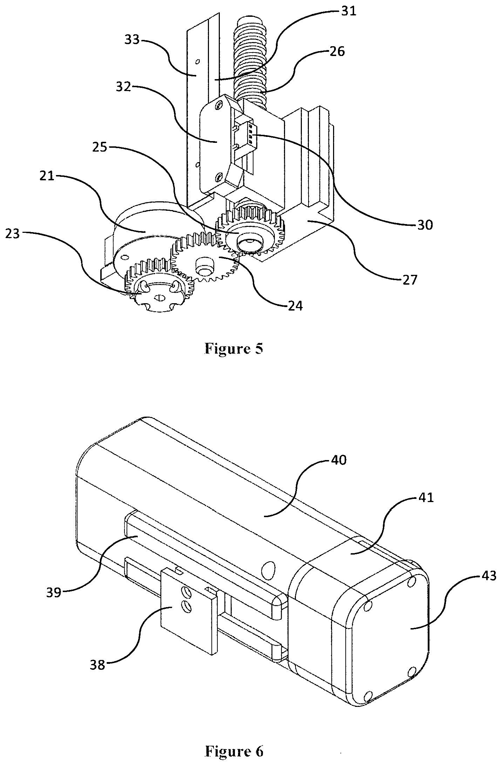

Fig.5 Y-stage mechanism with the electronic components for motion accuracy and actuation of the robotic system.
Fig.6 X-stage of the robotic system that carries the Θ-stage.

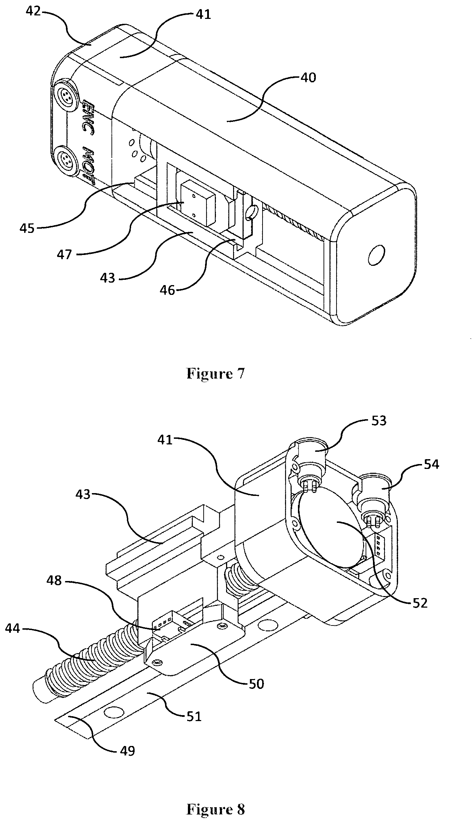

Fig.7 X-stage with coupling mechanism for the Y-stage.
Fig.8 X-stage mechanism and electronic components for motion accuracy and actuation of the robotic system.

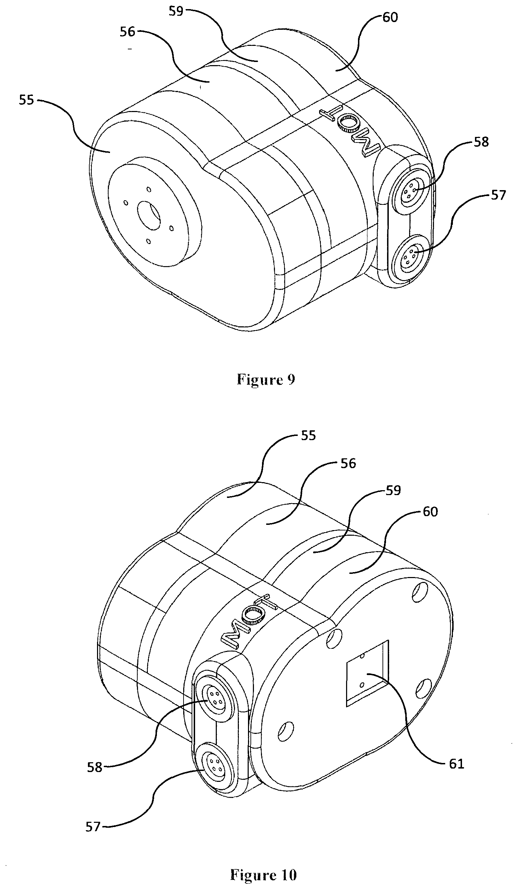

Fig.9 Θ-stage of the robotic system that angulates the transducer arm.
Fig. 10 Θ-stage with coupling for the X-stage (rear view).

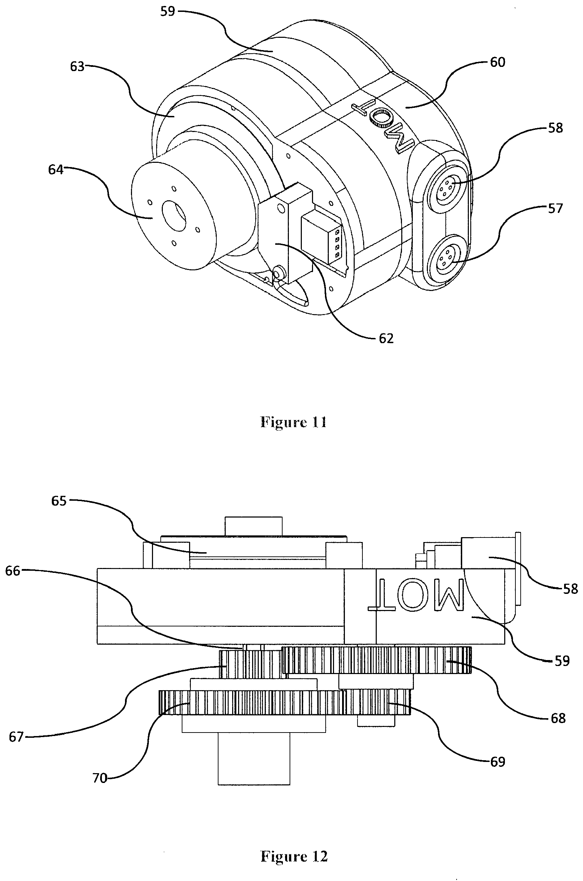

Fig.11 Angular encoder mechanism of the Θ-stage.
Fig.12 Gear speed reduction mechanism of the Θ-stage.

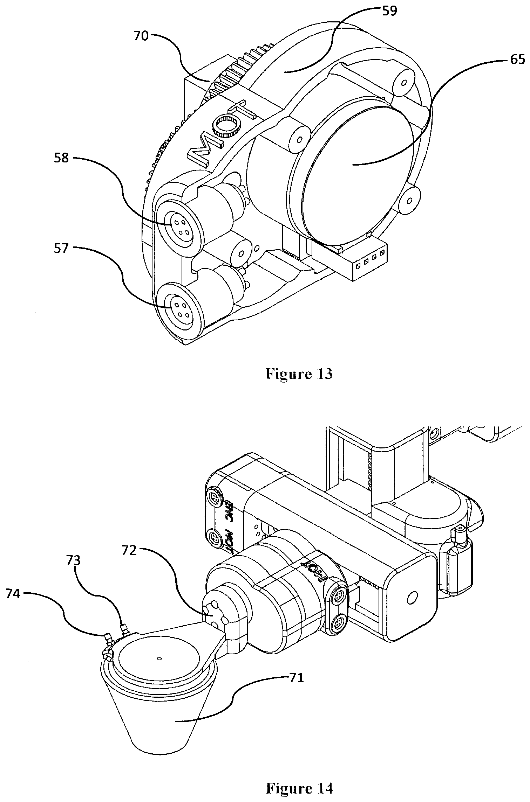

Fig.13 Θ-motor and encoder cabling of the Θ-stage.
Fig.14 Ultrasonic transducer as attached to the Θ-stage.

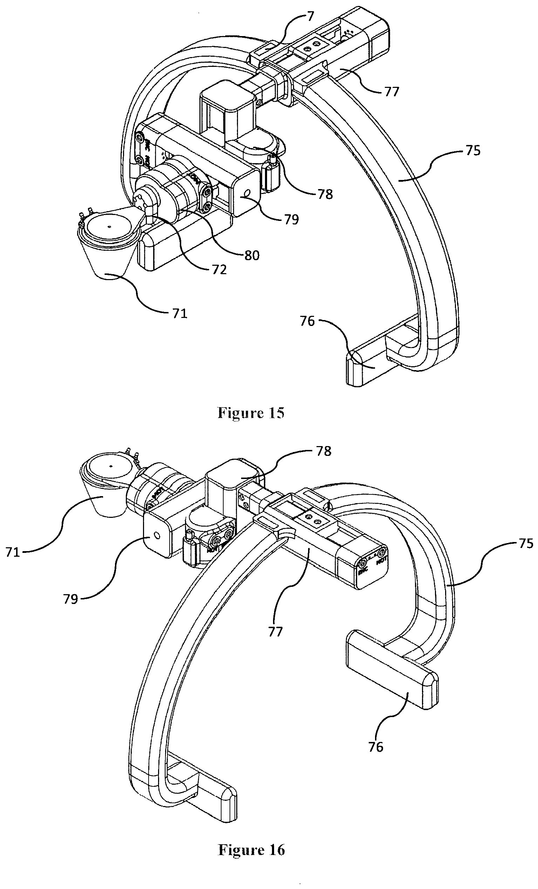

Fig.15 Robotic system attached to the c-arm.
Fig.16 Rear view of the robotic system attached to the c-arm.

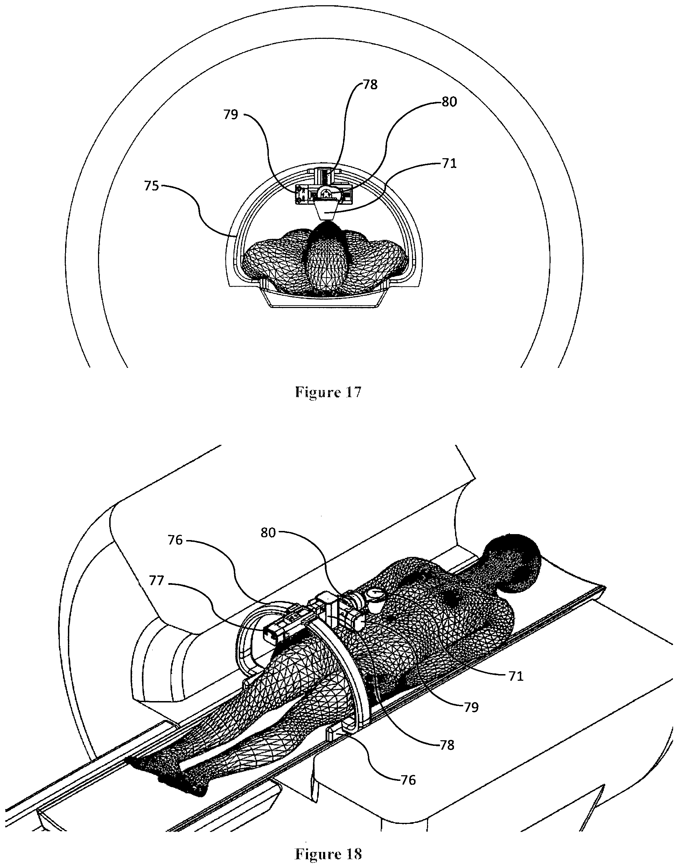

Fig.17 Robotic system and patient placed in the MRI scanner.
Fig.18 Robotic system with the patient inside the MRI scanner.

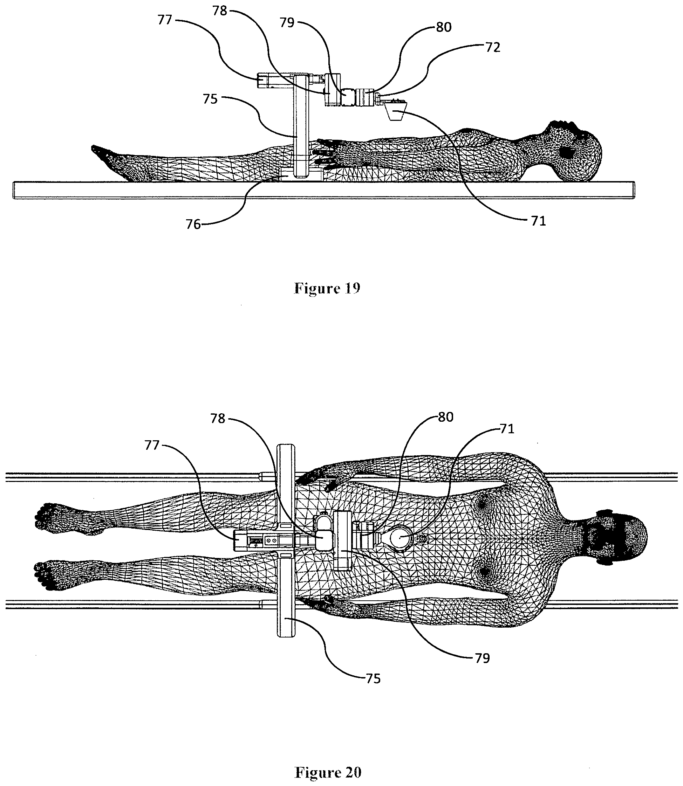

Fig.19 Side view of the robotic system as attached to the patient.
Fig.20 Top view of the robotic system as attached to the patient.

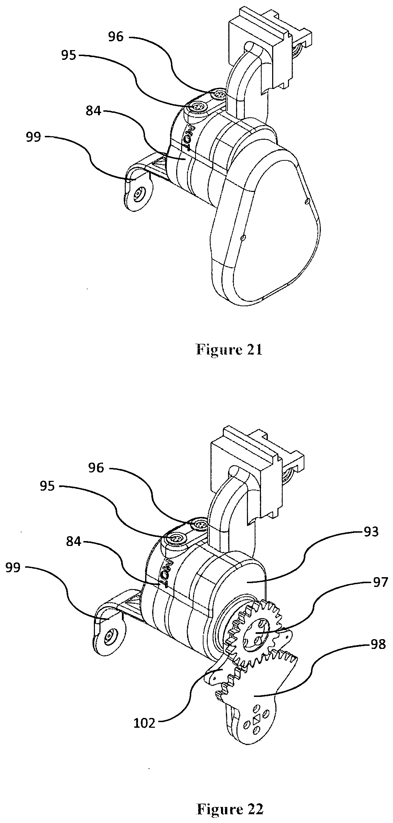

Fig.21 Φ-axis of the robotic system that carries the prostate device.
Fig.22 Φ-axis stage with the output gear and half gear.

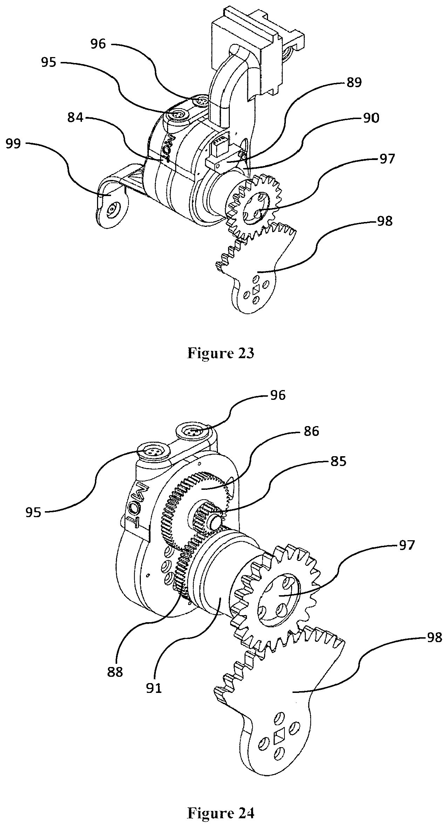

Fig.23 Angular encoder mechanism of the Φ-stage.
Fig.24 Gear speed reduction mechanism of the Φ-stage.

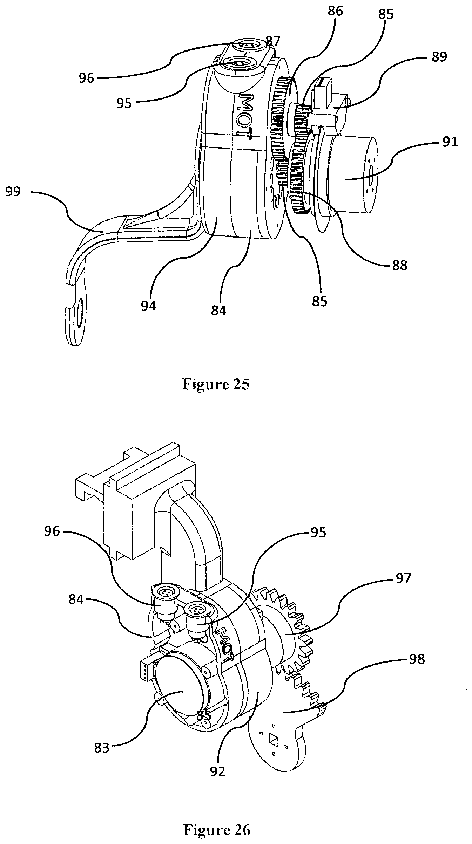

Fig.25 Gear speed reduction unit and encoder mechanisms of the Φ-stage.
Fig.26 Motor and encoder of the Φ-stage.

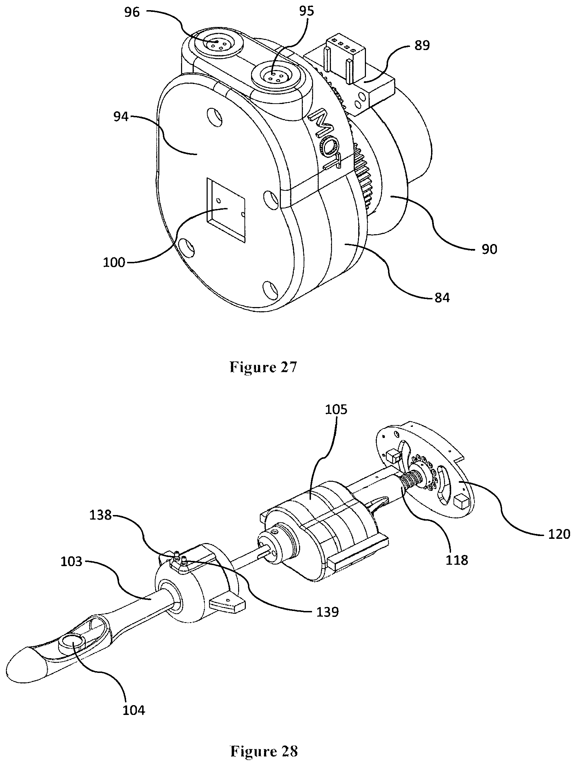

Fig.27 Rear view of the Φ-stage with the rectangular coupling.
Fig.28 Front view of the prostate robot mechanism.

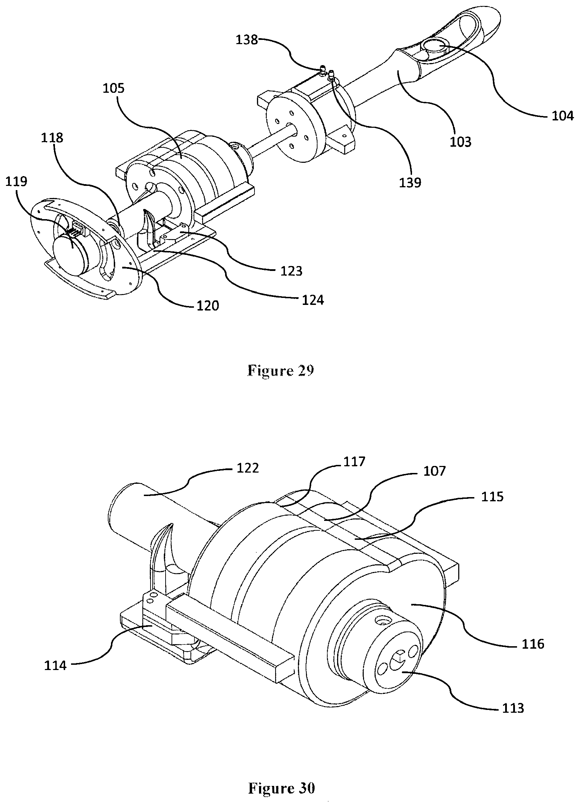

Fig.29 Rear view of the prostate robot mechanism.
Fig.30 Ψ-stage of the prostate robot.

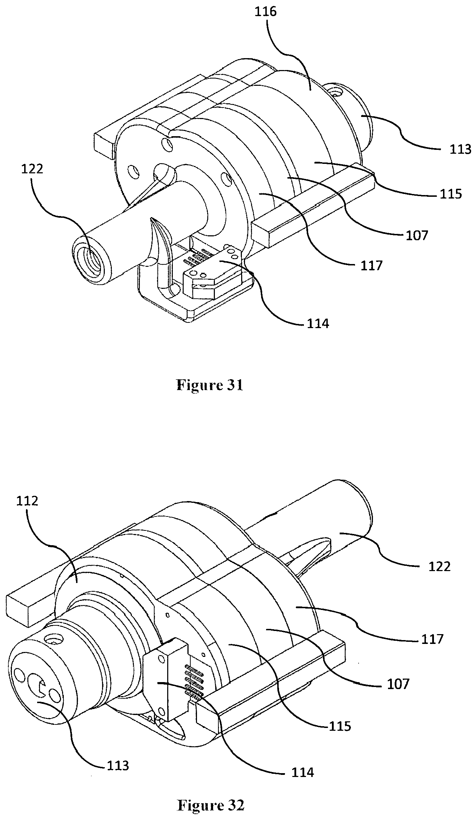

Fig.31 Ψ-stage rear view of the prostate robot with the ZZ-stage encoder module.
Fig.32 Angular encoder mechanism of the Ψ-stage.

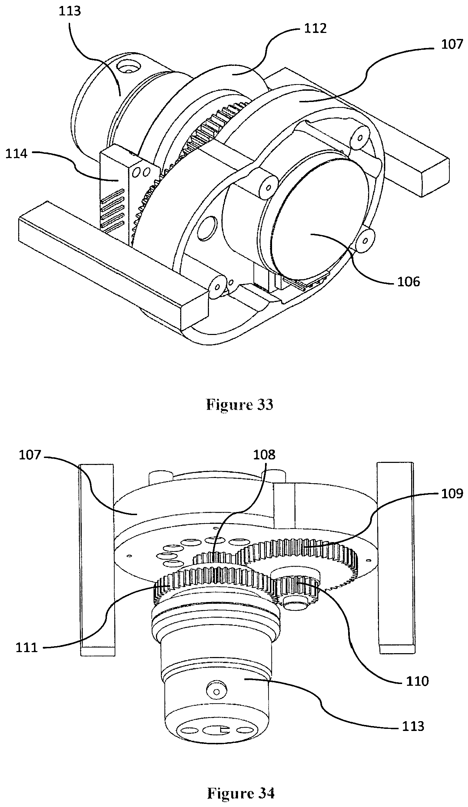

Fig.33 Φ-motor and encoder mechanism of the Ψ-stage.
Fig.34 Gear speed reduction unit with the encoder mechanism of the Ψ-stage.

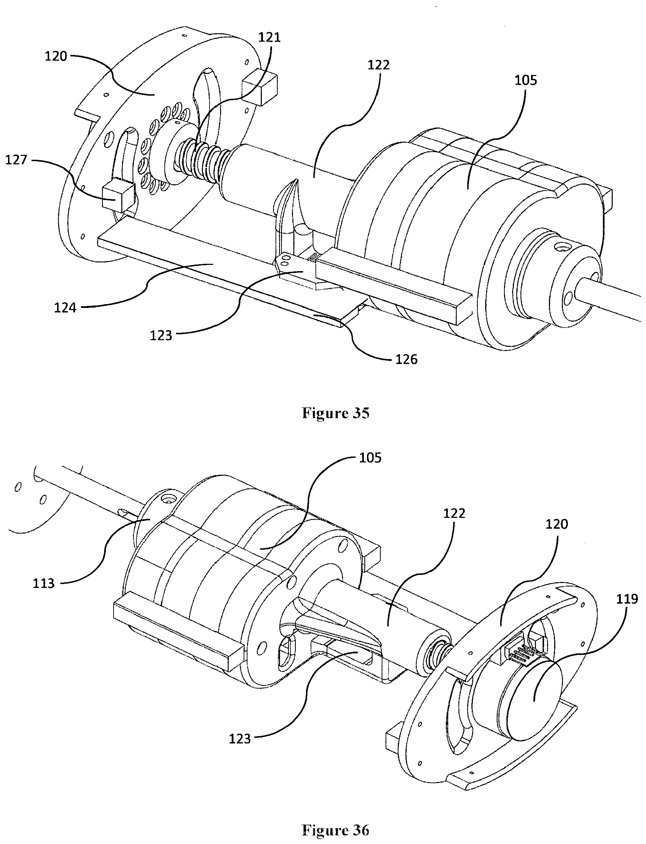

Fig.35 Ψ-stage and ZZ-stage of the prostate robot.
Fig.36 Ψ-stage and ZZ-stage of the prostate robot showing the ZZ-motor.

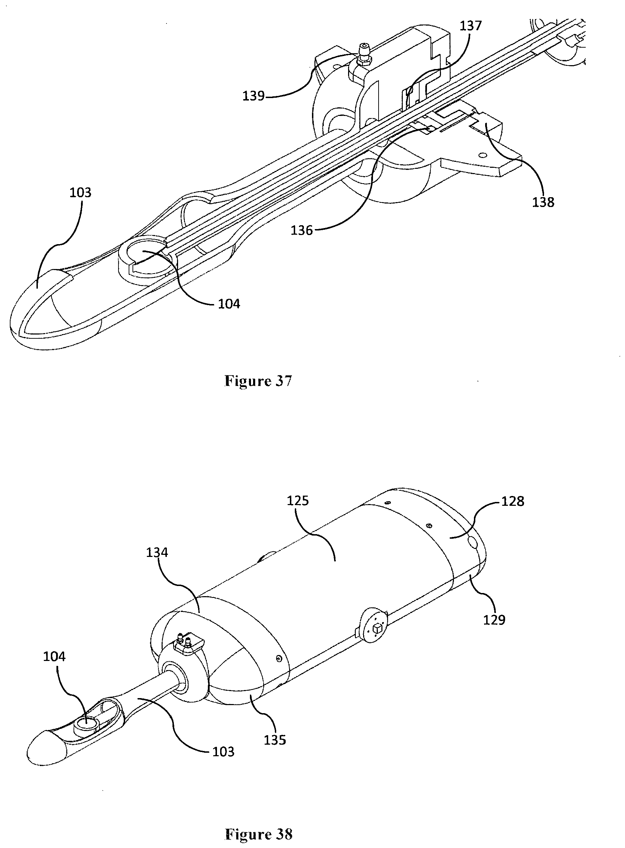

Fig.37 Endorectal probe cross section with the prostate transducer.
Fig.38 Endorectal probe attached to the prostate robot.

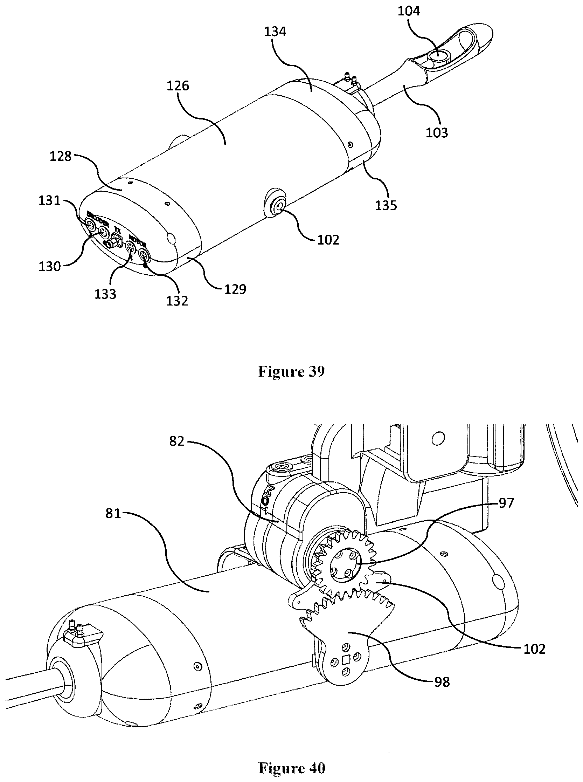

Fig.39 Prostate robot showing the connectors of the motors and encoders.
Fig.40 Φ-stage output gear coupled with the half gear.

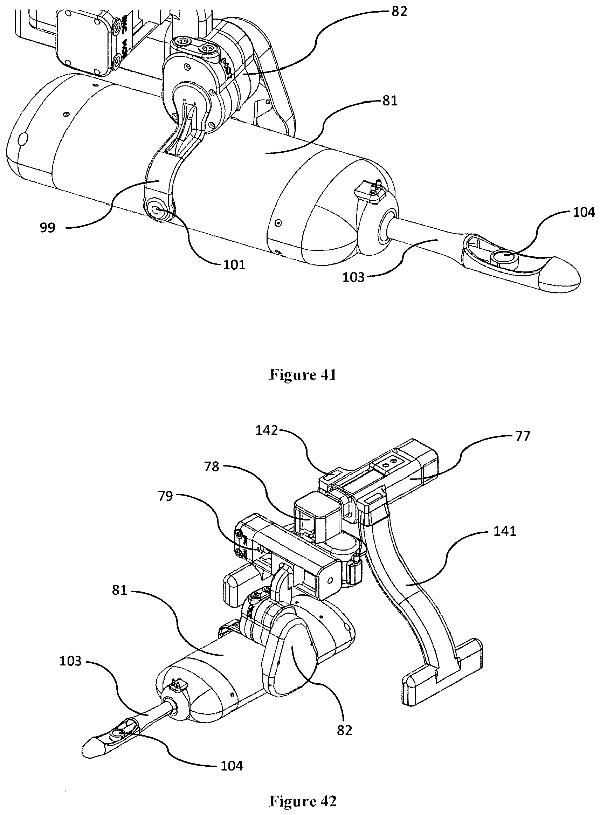

Fig.41 Prostate robot attached to the Φ-axis.
Fig.42 Prostate robot attached to the Φ-stage with the s-arms (front view).

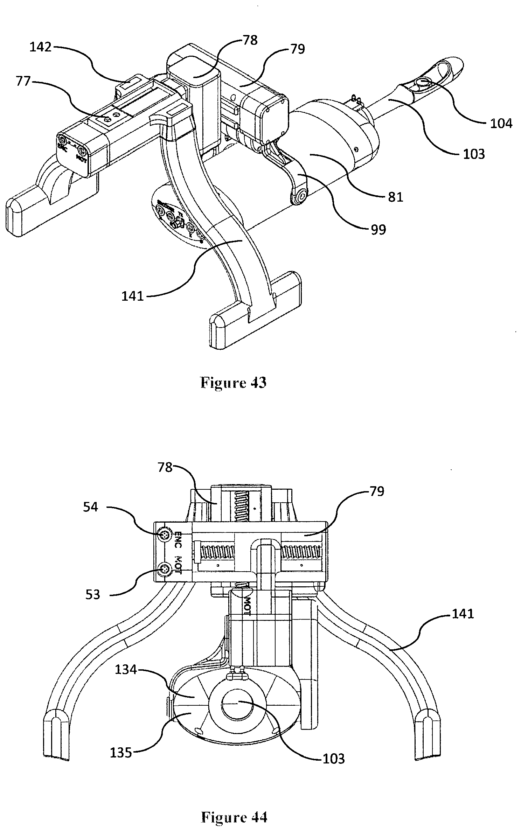

Fig.43 Prostate robot attached to the Φ-stage with the s-arms (rear view).
Fig.44 Front view of the device attached on the S-arms.

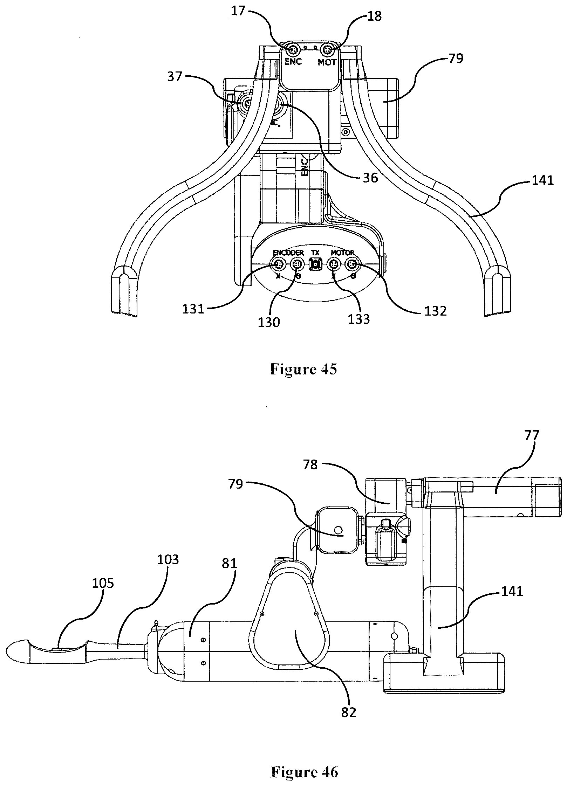

Fig.45 Rear view of the device attached on the S-arms.
Fig.46 Side view of the prostate robot.

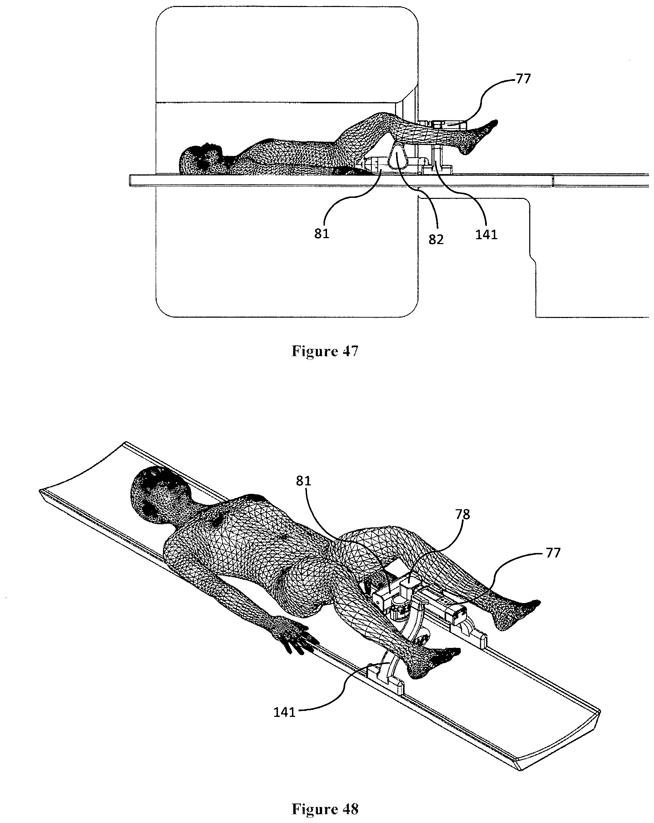

Fig.47 Prostate robot with the patient placed in the MRI magnet.
Fig.48 Prostate robot with the patient placed on the MRI table (rear view).

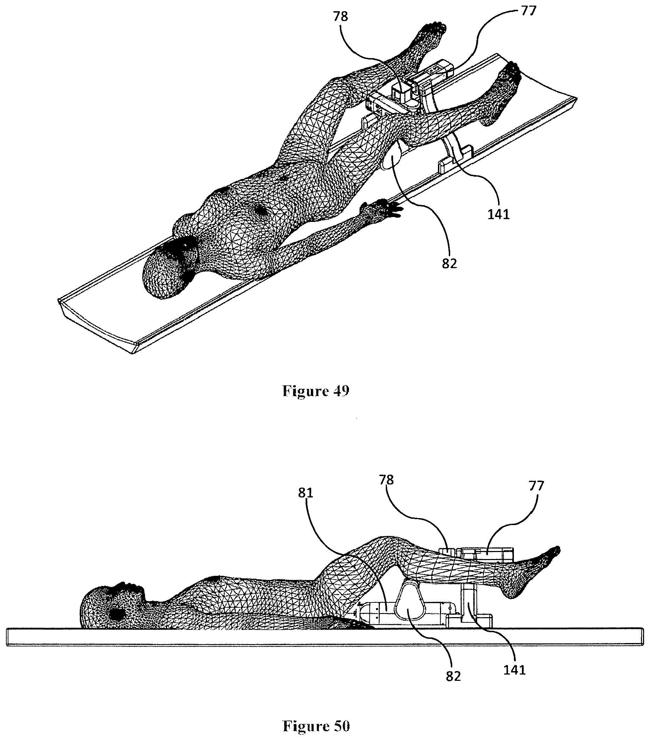

Fig.49 Prostate robot with the patient placed on the MRI table (front view).
Fig.50 Prostate robot with the patient placed on the MRI table (side view).

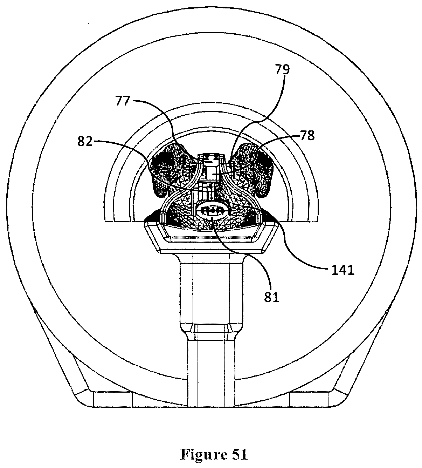

Fig.51 Prostate robot with the patient placed in the MRI magnet (rear view).

### DESCRIPTION OF AT LEAST ONE WAY OF CARRYING OUT THE INVENTION

The Z-plate 1 is placed in the guides 2 which are included in the inner side of the Z-frame 3. The Z-jack screw 4 is coupled to the Z-plate 1 to convert the angular motion to linear. The Z-motor holder 5 and the Z-motor cover 6 are attached to the Z-frame 3. On each side of the Z-frame 3 there are two couplings 7. The couplings 7 are used to attach the device to the MRI bed via two C-arms. The back of the Z-rectangular shaft 8 is fixed to the Z-plate 1 and the front includes a coupling 9. The Z-rectangular shaft 8 moves through the front guide 10 placed on the Z-frame 3 for smooth linear motion.

The Z-motor 11 is fixed on the Z-motor holder 5 and is covered by the Z-motor cover 6 which is also attached to the Z-motor holder 5. The Z-motor holder 5 is attached to the Z-frame 3 by the couplings 12 included on the Z-motor holder 5. The accuracy of the Z-stage is achieved with the use of an optical encoder module 13 and an encoder strip 14. The optical encoder module 13 is secured to the Z-plate 1 by the Z-encoder module holder 15. The encoder strip 14 is fixed to the Z-frame 3 by the Z-encoder strip holder 16. The Z-encoder connector 17 and the Z-motor connector 18 are placed on the back of the device and fixed between the Z-motor holder 5 and the Z-motor cover 6. The Z-encoder connector 17 and Z-motor connector 18 allow easy attachment and removal of the control cables.

The Y-frame 19 includes a coupling 20 that attaches to the Z-rectangular shaft 8 and is carried by the Z-stage. The Y-motor 21 attaches to the Y-motor holder 22 that is extended on the side of the Y-frame 19. On the Y-motor 21 attaches the Y-pinion gear 23 which is coupled to the Y-idler gear 24. The Y-idler gear 24 is coupled to the Y-spur gear 25 which rotates the Y-jack screw 26. The Y-jack screw 26 is coupled to the Y-plate 27 converting the angular motion to linear. On the top of the Y-plate 27 there are two slots 28 that attaches the X-stage. The Y-plate 27 is placed in the Y-frame guides 29 of the Y-frame 19. The Y-plate 27 is coupled to the Y-jack screw 26 converting the angular motion to linear.

The motion accuracy of the Y-stage is achieved the same way as the Z-stage. The Y-encoder module 30 counts the lines of the Y-encoder strip 31. The Y-encoder module 30 is fixed to the Y-plate 27 by the Y-encoder module holder 32. The Y-encoder strip 31 is held to the Y-frame 19 by the Y-encoder strip holder 33. The Y-motor 21 is covered by the Y-lower motor cover 34 and the Y-top motor cover 35. The Y-encoder connector 36 and the Y-motor connector 37, are secured between the motor covers.

The X-stage is mounted to the Y-stage by the X-Y stage coupling 38. The one side of the X-Y stage coupling 38 is attached to the Y-plate 27 and the other side is fixed to the mounting guides 39 on the rear of the X-frame 40. On the X-frame 40 the X-motor holder 41 and the X-motor cover 42 is attached.

The X-stage motion mechanism is the same as the Z stage and Y stage. The X-plate 43 is coupled to the X-jack screw 44 and moves within the guides 45 of the X-frame 40. On the top of the X-plate 43 there are guides 46 to mount the theta-stage coupling 47.

The motion accuracy is achieved by the X-encoder module 48 and the X-encoder strip 49 which measures the motion of the X-plate 43. The X-encoder module 48 is fixed to the X-plate 43 by the X-encoder module holder 50. The X-encoder strip 49 is pressed on the X-frame 40 by the X-encoder strip holder 51. The X-motor 52 is attached to the X-motor holder 41 and the X-motor holder 41 which is fixed to the X-frame 40. The X-motor connector 53 and the X-encoder connector 54 are secured by the X-motor holder 41 and the X-motor cover 42.

The Θ-stage encoder assembly is enclosed between the Θ-encoder cover 55 and in the Θ-gear cover 56. The Θ-encoder connector 57 and Θ-motor connector 58 are fixed laterally to the Θ-stage by the Θ-motor holder 59 and the Θ-motor cover 60. On the back of the Θ-motor cover 60 a female coupling 61 is used to connect the Θ-stage to the X-stage.

The motion accuracy of the Θ-stage is established by the Θ-encoder module 62 which measures the angular movement of the Θ-encoder disc 63. The Θ-encoder disk 63 is fixed below the Θ-encoder disk holder 64. The Θ-motor 65 is attached to the Θ-motor holder 59. On the motor shaft 66 the Θ-pinion gear 67 for the first stage of the speed reduction gear mechanism is fixed. The Θ-pinion gear 67 is coupled to the Θ-spur gear 68. The Θ-spur gear 68 includes the Θ-second pinion gear 69 which to rotates on the same axis, to transmit the motion to the second stage of the speed reduction gear mechanism. The Θ-second pinion gear 69 is coupled with the Θ-output gear 70 where the Θ-encoder disk 63 is also attached. The speed reduction gear mechanism increases the torque generated by the Θ-motor 65 to reduce the stress from the weight of the ultrasonic transducer 71 which is filled with degassed water. The gear ratio of each stage is 1:3, therefore the final gear ratio is 1:9.

On the front of the Θ-encoder disc holder 64 the Θ-arm 72 is attached that carries the ultrasonic transducer 71. The ultrasonic transducer 71 has a cone that is filled with degassed water that creates an acoustic coupling. On the top of the ultrasonic transducer 71 the inlet brass fitting 73 and the outlet brass fitting 74 are fasten. The inlet brass fitting 73 connects a hose to supply degassed water in the ultrasonic transducer 71 and the outlet brass fitting 74 allows the water to circulate for cooling.

The robotic system is attached on the two C-arms 75 via couplings 7. Each C-arm 75 is attached to the MRI table by two screws on the C-arm base 76. The Z-stage 77 is attached on the C-arms 75 and it carries the Y-stage 78. On the Y-stage 78 the X-stage 79 is attached which carries the Θ-stage 80. The Θ-stage 80 maneuvers the ultrasonic transducer 71 that is fixed to the Θ-arm 72. The robotic system is placed on the MRI table with the patient laying in the supine position.

The Φ-stage replaces the Θ-stage 80 that supports the prostate robot 81. The Φ-stage 82 uses the same mechanism as the Θ-stage 80, but the rotation axis is oriented parallel to the Y-stage 78. The Φ-motor 83 attaches to the Φ-motor holder 84 and couples to the Φ-pinion gear 85. The Φ-pinion gear 85 couples to the Φ-spur gear 86. The Φ-spur gear 86 includes the Φ-second pinion gear 87 which is coupled to the Φ-output gear 88. The rotation of the Φ-output gear 88 is monitored by the Φ-encoder module 89, that measures the movement of the Φ-encoder disk 90. The Φ-encoder disk 90 is fixed on the Φ-output gear 88 by the Φ-encoder disk holder 91. The Φ-encoder module 89 fastens on the Φ-gear cover 92. The encoder assembly is enclosed behind the Φ-encoder cover 93 for protection. On the back of the Φ-stage 82 the Φ-motor cover 94 is attached that holds the Φ-motor connector 95 and the Φ-encoder connector 96. The Φ-encoder disk holder 91 fastens on the Φ-output gear 97 and is coupled to the Φ-half gear 98. The Φ-half gear 98 connects to the left side of the prostate robot 81 via a coupling and screws. The right side of the prostate robot 81 is supported by the Φ-stage bracket 99. The Φ-stage bracket 99 is connected to the Φ-stage 82 by a rectangular coupling 100 and on the opposite side there is a hole which acts like a bearing around the Φ-stage pivot 101. The Φ-encoder cover 93 includes a bracket 102 which supports the right side of the prostate robot 81, creating a pivot joint behind Φ-half gear 98. The Φ-stage 82 tilts the prostate robot 81 to set the insertion angle of the endorectal probe 103.

The prostate robot 81 includes two axes to maneuver the prostate transducer 104. The prostate transducer 104 connects to the Ψ-stage 105 to angulate the ultrasonic beam inside the endorectal probe 103. The Ψ-stage 103 mechanism operates similarly with the Φ-stage 82. The Ψ-motor 106 attaches to Ψ-motor holder 107 and couples to the Ψ-pinion gear 108. Ψ-pinion gear 108 rotates the Ψ-spur gear 109 which includes the Ψ-second pinion gear 110. The Ψ-second pinion gear 110 rotates the Ψ-output gear 111. On the Ψ-output gear 111, the Ψ-encoder disk 112 is held by the Ψ-encoder disk holder 113. The motion of the Ψ-encoder disk 112 is measured by the Ψ-encoder module 114 which attaches to the Ψ-gear cover 115. The Ψ-encoder disk 112 and Ψ-encoder module 113 are protected by the Ψ-encoder cover 116. On the Ψ-encoder disk holder 113 the prostate transducer 104 is attached. The Ψ-motor 106 is enclosed by the Ψ-motor cover 117 and the Ψ-motor holder 107.

The Ψ-stage 105 is carried by the ZZ-stage 118, thus moving the Ψ-stage 105 forward and backwards. The ZZ-motor 119 is attached to the ZZ-motor holder 120. The ZZ-jack screw 121 attaches to the ZZ-motor 122 and couples to the Ψ-motor cover 117. Under the screw coupling 122 of the Ψ-motor cover 117 the ZZ-encoder module 123 is attached. The ZZ-encoder module 123 operates in conjunction with the ZZ-encoder strip 124 that is secured inside the prostate frame 125 by the ZZ-encoder strip holder 126. The ZZ-motor holder 120 fastens to the back of the prostate frame 125. The ZZ-motor holder 120 includes couplings 127 for the prostate rear-top cover 128 and the prostate rear-bottom cover 129. The prostate covers hold the Ψ-encoder connector 130, the ZZ-encoder connector 131, the Ψ-motor connector 132 and the ZZ-motor connector 133. On the front of the prostate frame 125 four structures support the prostate front-top cover 134 and the prostate front-bottom cover 135. The front prostate covers hold the endorectal probe 103 to the prostate robot 81.

Inside the endorectal probe 103 the prostate transducer 104 is located. The prostate transducer 104 maneuvering is achieved with Ψ-stage 105 and ZZ-stage 118. The endorectal probe 103 is covered by a condom and filled with degassed water to enable ultrasound to reach the patient. On the back of the endorectal probe 103, an outer O-ring 136 and an inner O-ring 137 prevent the leakage of the degassed water. The O-rings are held by the endorectal probe cover 138, that attaches to the rear of the endorectal probe 103. The inlet brass fitting 139 is used to supply the probe with degassed water and the outlet brass fitting 140 is used for the return of the degassed water back to the supply container. The degassed water circulation cools the prostate transducer 104 by supplying cold water.

The prostate robot 81 attaches to the MRI table by two S arms 141. The S-arms 141 have "S" shape to allow room for the patient legs. The S-arms 141 are shorter than the C-arms 75 to keep the probe closer to the MRI table, setting the appropriate insertion height of the endorectal probe 103. The S-arms 141 attaches to the bed with four screws (two on each arm). On the top of the S-arms 141 the Z-stage is attached via couplings 142. The patient lays in the MRI table in the supine position with the legs raised. The endorectal probe 104 is inserted into the patient's rectum to deliver the treatment.

### REFERENCES

[1]. American Cancer Society. Prostate Cancer Facts. In: Cancer Facts and Figures 2009. Atlanta, GA: American Cancer Society,2009; 19-20.
[2]. European Cancer Observatory. Cancer Fact Sheets. Available from: URL: http://eu-cancer.iarc.fr/2-cancer-factsheets.html,en.
[3]. Warmuth M, et. al. Systematic review of the efficacy and safety of high-intensity focussed ultrasound for the primary and salvage treatment of prostate cancer. Eur Urol 2010; 58: 803-815.
[4]. Ananthakrishnan A., Gogineni V., Saeian K., Epidemiology of Primary and Secondary Liver Cancers, Semin Ontervent Radiol., 2006, Mar; 23(1): 47-63
[5]. World Health Organization, I.A.f.R.o.C, Estimated Cancer Incidence, Mortality and Prevalence worldwide in 2018. Available from: http://pco.iarc.fr/today/data/factsheets/cancers/11-Liver-fact-sheet.pdf
[6]. Ghouri Y.A., Mian I., Rowe J.H., Review of hepatocellular carcinoma: Epidemiology, etiology and carcinogenesis, Journal of Carcinogenesis, 2017, 16(1)
[7]. Ljungberg B., Campbell S.C., Choi H.Y., Jacqmin D., Lee J.E., Weikert S., Kiemeney L.A., The epidemiology of Renal Cell Carcinoma, European Urology, 2011, Oct; 60(4): 615-621
[8]. World Health Organization, I.A.f.R.o.C, Estimated Cancer Incidence, Mortality and Prevalence worldwide in 2018. Available from: http://gco.iarc.fr/todav/data/factsheets/cancers/29-Kidney-fact-sheet.pdf
[9]. Chow W.H., Dong L.M., Devesa S.S., Epidemiology and risk factors for kidney cancer, Nat. Rev. Urology, 2010, May; 7 (5):245-257
[10]. Ilic M., Ilic I., Epidemiology of pancreatic cancer, World Journal of Gastroenterology, 2016 Nov 28; 22(44): 9694-9705
[11]. World Health Organization, I.A.f.R.o.C, Estimated Cancer Incidence, Mortality and Prevalence worldwide in 2018. Available from: http://gco.iarc.fr/today/data/factsheets/cancers/13-Pancreas-fact-sheet.pdf
[12]. Ghadirian P., Lynch H.T., Krewski D., Epidemiology of pancreatic cancer: an overview, Cancer Detection and Prevention, 2003, 27(2): 87-93
[13]. American Urological Association Education and Research, Inc. Guidelines for the management of clinically localized prostate cancer: 2007 update. Available from: URL: http://www.auanet.org/content/clinical-practice-guidelines/clinical-guidelines/main-reports/proscan07/appendixes.pdf.
[14]. Heidenreich A, et. al. Guidelines on Prostate Cancer. January 2011 update. Available from: URL: http://www.uroweb.org/guidelines/online-guidelines/.
[15]. Bill-Axelson et. al. Radical prostatectomy versus watchful waiting in localized prostate cancer: the Scandinavian prostate cancer group-4 randomizedtrial. J Natl Cancer Inst 2008; 100: 1144-1154.
[16]. Lukka H, et. al. High-intensity focused ultrasound for prostate cancer: a systematic review. Clin Oncol (R Coll Radiol) 2011; 23: 117-127
[17]. Ahmed, H. U. The index lesion and the origin of prostate cancer. N. Engl. J. Med. 361, 1704-1706 (2009).
[18].http://innovativecancersolutions.hk/italian-urologistsrecommends-hifu.html.
[19]. Jolesz FA. MRI-guided focused ultrasound surgery. AnnuRev Med 2009; 60: 417-430.
[20]. Dick EA, Gedroyc WM. Expert Rev Med Devices 2010; 7: 589-597.
[21]. Blana A, et. al. Eight years' experience with highintensity focused ultrasonography for treatment of localized prostate cancer. Urology 2008; 72: 1329-1333; Discussion 1333-1334
[22]. Murat FJ, et. al. A. Eur Urol 2009; 55:640-647.
[23]. Murat FJ, et. al. Prognostic factors for salvage HIFU success after external beam radiation failure (EBRT) failure. Eur Urol Suppl 2008; 7: 119.
[24]. Rebillard X, et. al. High-intensity focused ultrasound in prostate cancer; a systematic literature review of the French Association of Urology. BJU Int 2008; 101: 1205-1213.
[25]. National Institute for Health and Clinical Excellence (NICE). IPG118 High-intensity focused ultrasound for prostate cancer: guidance. Available from: URL: http://guidance. nice.org.uk/IPG118/Guidance/pdf.
[26]. Napoli A, et. al. Clinical Foci in Interventional Oncology. Code VO41-16.RSNA 2010
[27]. Cheng CWS, et. al. Initial experience with MRg FUS focal therapy for low risk prostate cancer. European Congress of Radiology 2011; Mar 3-7; Vienna, Austria.
[28]. Siddiqui et. al. MRI-guided transurethral ultrasound therapy of the prostate gland using real-time thermal mapping: initial studies. Urology 2010; 76:1506-1511.
[29]. Chopra R, et. al. Analysis of the spatial and temporal accuracy of heating in the prostate gland using transurethral ultrasound therapy and active MR temperature feedback.Phys Med Biol 2009; 54: 2615-2633.
[30]. Hazle JD, et. al. MRI-guided thermal therapy of transplanted tumors in the canine prostate using a directional transurethral ultrasound applicator. J Magn Reson Imaging 2002; 15:409-417
[31]. Pauly KB, et. al. Magnetic resonanceguided high-intensity ultrasound ablation of the prostate. Top Magn Reson Imaging 2006; 17: 195-207.
[32]. Starlz T.E., Marchioro T.L., Kaulla K.N., Hermann G., Brittain R.S., Waddell W.R., Homotransplantation of the liver in humans, Surg. Gynegol. Obstet, 1963 Dec;117:659-676
[33]. Lu W., Dong J., Hepatectomy of hepatocellular carcinoma in the era of liver transplantation, World Journal of Gastroenterology, 2014, Jul; 20(28): 9237-9244
[34]. Masaaki E., Shinchiro O., Kazuhiko K., Nobuyuki S., Hiroyuki F., Masaharu Y., Fukuo K., Hiromitsu S., Percutaneous ethanol injection for small hepatocellular carcinoma: Therapeutic efficacy based on 20-year observation, Journal of hepatology, 2005, Sep; 43(3):458-464
[35]. Ansari D., Andersson R., Radiofrequency ablation of percutaneous ethanol injection for the treatment of live tumours, World Journal of Gastroenterology, 2012, Mar; 18(10):1003-1008
[36]. Friedman M., Mikityansky I., Kam A., Libutti S.K., McCellan M.W., Neeman Z., Locklin J.K., Wood B.J., Radiofrequency ablation of cancer, Cardiovasc. Intervent Radiol., 2004 ;27 (5):427-434
[37]. Dick E.A., Taylor-Robinson S.D., Thomas H.C., Gedroyc W.M.W., Ablative therapy for liver tumours, Gut., 2002 May; 50(5): 733-739
[38]. Frizzell L., Linke C., Carstensen E., Fridd C., Thresholds for focal ultrasound lesions in rabbit kidney, liver and testicle, IEEE Transactions on Biomedical Engineering, 1987, 24(4):393-396
[39]. Seket B., Lafon C., Salomir R., Chapelon J.Y., Paquet C., Scoazec J.Y., Cathignol D., Morphological Analysis of the Interstitial Ultrasonic Ablation in Porcine Liver in vivo, Eur. Surg. Res., 2008; 41:24-32
[40]. Chen L., Rivens I., Haar G., Riddler S., Hill C.R., Bensted J.P.M., Histological changes in rat liver tumours treated with high-intensity focused ultrasound, Ultrasound in Med. & Biol., 1993, 19(1):67-74
[41]. Wijlemans J.W., Bartels L.W., Deckers R., Ries M., Mali W.P.Th.M., Moonen C.T.W., Bosch M.A.A.J., Magnetic resonance-guided high-intensity focused ultrasound (MR-HIFU) ablation of liver tumours, Cancer Imaging, 2012 ; 12(2): 387-394
[42]. Kopelman D., Inbar Y., Hanannel A., Freundlich D., Castel D., Perel A., Greenfeld A., Salamon T., Sareli M., Valeanu A., Papa M., Magnetic resonance-guided focused ultrasound surgery (MRgFUS): Ablation of liver tissue in a porcine model, European Journal of Radiology, 2006; 59: 157-162
[43]. Courivaud F., Kazaryan A.M., Lund A., Orszagh V.C., Svindland A., Marangos I.P., Halvorsen S., Jebsen P., Fosse E., Hol K., Edwin B., Thermal. Fixation of swine liver tissue after magnetic resonance-guided high-intensity focused ultrasound ablation, Ultrasound in Med & Biol, 2014; 40(7):1564-1577
[44]. Jiang F., He M., Liu Y.J., Wang Z.B., Zhang L., Bai J., High intensity focused ultrasound ablation of goat liver in vivo: Pathologic changes of portal vein and the "heat-sink" effect, Ultrasonics, 2013; 53:77-83
[45]. Chuan-Xing L., Xu G., Jiang Z., Li J., Luo G., Shan H., Zhang R., Yin L., Analysis of clinical effect of high-intensity focused ultrasound on liver cancer, World Journal of Gastroenterology, 2004; 10(15):2201-2204
[46]. Wu F., Wang Z., Chen W., Zhu H., Bai J., Zou J., Li K., Jin C., Xie F., Su H., Extracorporeal High intensity focused ultrasound ablation in the treatment of patients with large hepatocellular carcinoma, Annals of Surgical Oncology, 2004; 11(12): 1061-1069
[47]. Fukuda H., Numata K., Nozaki A., Morimoti M., Kondo M., Tanaka K., Maeda S., Ohto M., Ito R., Zhu H., Wang Z., Findings of multidetector row computed tomography of HCCs treated by HIFU ablation, European Journal of Radiology, 2011, doi:10.1016/j.ejrad.2011.01.101
[48]. Xu G., Luo G., He L., Li J., Shan H., Zhang R., Li Y., Gao X., Lin S., Wang G., Follow-up of high-intensity focused ultrasound treatment for patients with hepatocellular carcinoma, Ultrasound in Med.&Biol., 2011; doi:10.1016/j.ultrasmedbio.2011.08.011
[49]. Zhang L., Zhu H., Zhou K., Li K., Su H., Chen W., Bai J., Wang Z., High-intensity focused ultrasound (HIFU): effective and safe therapy for hepatocellular carcinoma adjacent to major hepatic veins, Eur. Radiol, 2009; 19:437-445
[50]. Zhang Y., Zhao J., Guo D., Zhong W., Ran L., Evaluation of short-term response of high intensity focused ultrasound ablation for primary hepatic carcinoma: Utility of contrast-enhanced MRI and diffusion-weighted imaging, Eur. J. Radiol, 2010, doi:10.1016/j.ejrad.2010.06.039
[51]. Zhu H., Zhou K., Zhang L., Jin C., Peng S., Yang W., Li K., Su H., Chen W., Bai J., Wu F., Wang Z., High intensity focused ultrasound (HIFU) therapy for local treatment of hepatocellular carcinoma: Role of partial rib resection, European Journal of Radiology, 2009; 72:160-166
[52]. Senneville B.D., Moonen C., Ries M., MRI-Guided HIFU methods for the ablation of liver and renal cancers, Therapeutic ultrasound, Advances in experimental Medicine and Biology, 2016, vol 880., Springer, Cham
[53]. Chin A., Lam J.S., Figlin R.A., Beldegrun A.S., Surveillance strategies for renal cell carcinoma patients following nephrectomy, Rev. Urol., 2006; 8(1):1-7
[54]. Cozar J.M., Tallada M., Open partial nephrectomy in renal cancer: a feasible gold standard technique in all hospitals, Advances in Urology, 2008 : 916463
[55]. Minervini A., Ficcarra V., Rocco F., Antonelli A., Bertni R. et al., Simple enucleation is equivalent to traditional partial nephrectomy for renal cell carcinoma: results of a nonrandomized, retrospective, comparative study, J. Urol., 2011;185(5):1604-10
[56]. Adams J.B., Moore R.G., Anderson J.H., Strandberg D.V.M., Marshall J.D., Davoussi L.R., High-Intensity focused ultrasound ablation of rabbit kidney tumors, Journal of endourology, 1996; 10(1):71-75
[57]. Paterson R.F., Barret E., Siqueira T., Gardner T., Tavakkoli J., Rao V., Sanghvi N., Cheng L., Shalhav A., Laparoscopic partial kidney ablation with high intensity focused ultrasound, The Journal of Urology, 2003; 169 :347-351
[58]. Watkin N., Morris S., Rivens I., Haar G., High- intensity focused ultrasound ablation of the kidney in a large animal model, Journal of Endourology, 1997; 11(3):191-196
[59]. Hacker A., Michel M., Marlinghaus E., Kohrmann K., Alken P., Extracorporeally induced ablation of renal tissue by high-intensity focused ultrasound, BJU Int, 2006; 97:779-785
[60]. Vallancien G, Chartier-Kastler E, Harouni M, Chopin D, Bougaran J., Focused extracorporeal pyrotherapy: Experimental study and feasibility in man, Semin. Urol., 1993;11:7-9.
[61]. Wu F., Wang Z.B., Chen W., Bai J., Zhu H., Qiao T., Preliminary experience using high intensity focused ultrasound for the treatment of patients with advanced stage renal malignancy, Journal of Urology, 2003; 170(6 Pt 1):2237-40
[62]. Klingler C., Susani M., Seip R., Mauermann J., Sangvi N., Marberger M., A novel approach to energy ablative therapy of small renal tumours: Laparoscopic High-Intensity focused ultrasound, European Urology, 2008; 53:810-818
[63]. Marberger M., Schatzl G., Cranston D., Kennedy J., Extracorporeal ablation of renal tumours with high-intensity focused ultrasound, BJU , 2005; 95(2):52-55
[64]. Iling W., Kennedy J., Wu F., Haar G et al., The safety and feasibility of extracorporeal high-intensity focused ultrasound (HIFU) for the treatment of liver and kidney tumours in a western population, British Journal of Cancer, 2005; 93 (8):890-895
[65]. Saeed M., Krug R., Hetts S., Wilson M., Renal ablation using magnetic resonance-guided high intensity focused ultrasound: Magnetic resonance imaging and histopathology assessment, World Journal of Radiology, 2016; 8(3):298-307
[66]. Acher A., Bleicher J., Cannon A., Scaife C., Advances in surgery for pancreatic cancer, Journal of Gastrointestinal Oncology, 2018; 9(6):1037-1043
[67]. Springfeld C., Jager D., Buchler MW., Strobel O., Hackert T., Palmer DH, Neoptolemos JP, Chemotherapy for pancreatic cancer, Presse Med., 2019; 48(3 Pt 2):159-174
[68]. Hadjicostas P., Malakounides N., Varianos C., Kitiris E., Lerni F., Symeonides P., Radiofrequency ablation in pancreatic cancer, HPB, 2006; 8(1):61-64
[69]. Xie B., Li Y., Jia L., Nie., Du H., Jiang S., Experimental ablation of the pancreas with high intensity focused ultrasound (HIFU) in a porcine model, Int. Journal of Med. Sci., 2011; 8(1):9-15
[70]. Mao Y., Fang L., Ai L., Li C., Wang Z., Wu J., Bai J., Li F., An in vivo study of the effects on serum glucose, amylase and histopathology of the feline pancreatic tissue treated by focused ultrasound, Plos one, 2014; 9(2)
[71]. Liu C., Gao X., Xiong L., Ge H., He X., Li T. et al., A preclinical in vivo investigation of high- intensity focused ultrasound combined with radiotherapy, Ultrasound in Med.&Biol., 2011; 37(1):69-77
[72]. Jiang L., HuB., Guo Q., Chen L., Treatment of pancreatic cancer in a nude mouse model using high-intensity focused ultrasound, Experimental and therapeutic medicine, 2013; 5:39-44
[73]. Xie B., Ling J., Zhang W., Huang X., Zhen J., Huang Y., The efficacy of high-intensity focused ultrasound in advanced pancreatic cancer, Clin. Oncol. Cancer Res., 2008; 5:183-186
[74]. Atsushi S., Moriyasu F., Sano T., Itokawa F., Tsuchiya T. et al., Safety trial of high-intensity focused ultrasound therapy for pancreatic cancer, World journal of Gastroenterology, 2014; 20(28):9570-9577
[75]. Ge H., Miao L., Xiong L., Yan F. et al., High-intensity focused ultrasound treatment of late-stage pancreatic body carcinoma: optimal tumour depth for safe ablation, Ultrasound in Med.&Biol, 2014; 40(5):947-955
[76]. Li P., Zhu S., He W., Zhu L., Liu S., Liu Y., Wang G., Ye F., High-intensity focused ultrasound treatment for patients with unresectable pancreatic cancer, Hepatobiliary Pancreat. Dis. Int., 2012; 11(6):655-660
[77]. Wu F., Wang Z., Zhu H., Chen W., Zou J., Bai J., Li K., Jin C., Xie F., Su H., Feasibility of US-guided High-intensity focused ultrasound treatment in patients with advanced pancreatic cancer: Initial experience, Radiology, 2005; 236:1034-1040
[78]. Xiong L., Hwang J., Huang X., Yao S., He C., Ge X., Ge H., Wang X., Early clinical experience using high intensity focused ultrasound for palliation of inoperable pancreatic cancer, JOP. J. Pancreas, 2009; 10(2): 123-129
[79]. Lee J., Choi B., Ryu J., Kim Y., Hwang J., Kim H., Han J., Concurrent chemotherapy and pulsed high-intensity focused ultrasound therapy for treatment of unresectable pancreatic cancer: Initial experience, Korean Journal of Radiology, 2011; 12(2):176-186

## Claims

1. A positioning device for navigating an energy source under magnetic resonance imaging guidance for treating prostate abdominal, bone, chest and brain targets comprising:
A c-shape (70) holder that is attached to the MRI table using a few non-magnetic screws.

2. The C-shape holder of claim 1, wherein the first stage for supine access for moving the positioning device in the MRI Z axis (72) comprises:
a first piezoelectric motor (11);
a Z-axis moving plate (1);
a coupling that attaches to the C-frame (7);
a coupling that holds the Y-axis (9);
a holder (5) supporting the piezoelectric motor;
a first jack screw (4) coupled to the piezoelectric motor;
and an optical encoder (13) and encoder connector (17)

3. The Z stage of claim 2, wherein the second stage for supine access for moving the positioning device in the MRI Y axis (73) comprises:
a second piezoelectric motor (19);
a Y-axis moving plate (20);
a coupling that holds the X-axis (25);
a holder (20) supporting the piezoelectric motor;
a second jack screw (23) coupled to the piezoelectric motor;
and an optical encoder (26) and encoder connector (31)

4. The Y stage of claim 3, wherein the third stage for supine access for moving the positioning device in the MRI X axis (74) comprises:
a third piezoelectric motor (48);
an X-axis moving plate (39);
a coupling that holds the Θ-axis (43);
a holder (37) supporting the piezoelectric motor;
a third jack screw (41) coupled to the piezoelectric motor;
and an optical encoder (44) and encoder connector (50)

5. The X stage of claim 4, wherein the fourth stage for supine access for moving the positioning device in the MRI Θ axis (68) comprises:
a fourth piezoelectric motor (64);
a coupling that holds an energy source (68);
a holder (62) supporting the piezoelectric motor;
a pinion gear (64), a pair of speed reduction gears (66, 67);
and an optical encoder (58) and encoder connector (53).

6. The c-shape holder of claim 1, wherein the fifth stage for prostate access for moving the positioning device in the ϕ axis (82) comprises:
a fifth piezoelectric motor (83);
a Φ-pinion gear 85, a Φ-spur gear 86 and a Φ-half gear (98);
a Φ-second pinion gear 87 which is coupled to the Φ-output gear (88);
a holder (84) supporting the piezoelectric motor;
a Φ-stage bracket (99) which is attached to the prostate robot (81);
a coupling that attaches the ϕ stage to the X stage (79);
and an optical encoder (89) and encoder connector (96).

7. The ϕ axis of claim 6, wherein the sixth stage for prostate access for moving the positioning device in the Ψ axis comprises:
a sixth piezoelectric motor (106);
a Ψ -pinion gear (108), a Ψ-spur gear (109), a Ψ-second pinion gear (110) and a Ψ-output gear (111);
a prostate transducer (104);
a holder (107) supporting the piezoelectric motor;
and an optical encoder (113) and encoder connector (96).

8. The Ψ axis of claim 7, wherein the seventh stage for prostate access for moving the positioning device in the ZZ axis comprises:
a seventh piezoelectric motor (122);
a ZZ-jack screw (121) attaches to the ZZ-motor (122);
a prostate rear-top cover (128) and the prostate rear-bottom cover (129);
a holder (120) supporting the piezoelectric motor;
and an optical encoder (123).

9. The robotic system of claim 1 wherein in use the water container is filled with degassed water so that ultrasound energy is acoustically coupled to the target and wherein, in use, the FUS transducer (104) is immersed in the water container.

10. The robotic system of claim 8, further comprises an ultrasound transparent membrane attached to the water container and adapted for contact with the target.

11. The robotic system of claim 1 being placeable on the table of an MRI scanner adapted to access to a patient that is positioned in supine position.

12. The robotic system of claim 1, adapted to access targets from top to bottom for fibroids, brain, abdominal, bone, prostate and breast tumor targets.

13. The robotic device of claim 1, guidable by magnetic resonance imaging (MRI) comprising:
a magnetic resonance (MR) imaging means for imaging brain, bone, prostate, breast, fibroid, and
abdominal sites and for creating temperature sensitive images of ablated sites during surgery;

## Amended claims

### Amended claims in accordance with Rule 137(2) EPC.

1. A robotic device for navigating an energy source under magnetic resonance imaging guidance for access to multiple targets with a patient in supine position, comprising:
two C-arms (75);
a C-arm base (76) being adapted to connect said C-arms to any conventional MRI table;
a Z-stage (77) for movements along the Z-axis, wherein said Z-axis is increasing toward the head of the patient;
a Y-stage (78) for movements along the Y-axis, wherein said Y-axis is increasing to the posterior side of the patient;
a X-stage (79) for movements along the X-axis, wherein said X-axis is increasing to the left-hand side of the patient.

2. The robotic device of claim 1, wherein said Z-stage comprises:
a Z-axis moving plate (1);
a first piezoelectric motor (11);
a holder (5) supporting the piezoelectric motor;
a first jack screw (4) coupled to the piezoelectric motor;
a coupling (7) that attaches to said C-arms;
a coupling (9) that holds the Y-stage;
and an optical encoder module (13) and encoder connector (17).

3. The robotic device of claim 2, wherein said Y-stage comprises:
a Y-axis moving plate (27);
a second piezoelectric motor (21);
a holder (22) supporting the piezoelectric motor;
a second jack screw (26) coupled to the piezoelectric motor;
a series of gears (23,24,25) being adapted to enable attachment of said motor lateral to said Y-axis plate;
a coupling (38) that holds the X-stage;
and an optical encoder module (30) and encoder connector (36).

4. The robotic device of claim 3, wherein said X-stage comprises:
an X-axis moving plate (43);
a third piezoelectric motor (52);
a holder (41) supporting the piezoelectric motor;
a third jack screw (44) coupled to the piezoelectric motor;
and an optical encoder module (48) and encoder connector (54).

5. The robotic device of claim 4, wherein said X-stage further comprises a detachable Θ-stage (80), said Θ-stage being connected to said X-stage via coupling (61), and being adapted to enable rotation about a fixed axis parallel to said Z-axis.

6. The robotic device of claim 5, wherein said Θ-stage comprises:
a fourth piezoelectric motor (64 65);
a holder (59) supporting the piezoelectric motor;
a Θ-pinion gear (67), a Θ-second pinion gear (69);
a pair of speed reduction gears (68, 70);
and an optical encoder module (62) and encoder connector (57);
and a Θ-arm (72) being connected to said Θ-stage and being adapted to carry an ultrasonic transducer (71) in a cone container.

7. The robotic system of claim 4, wherein said X-stage further comprises a detachable Φ-stage (82) being adapted to enable rotation of a prostate module (81) about a fixed axis parallel to said X-axis, said Φ-stage comprises:
A fourth piezoelectric motor (83);
a holder (84) supporting the piezoelectric motor;
a Φ-pinion gear (85), a Φ-spur gear (86);
a Φ-second pinion gear (87) which is coupled to the Φ-output gear (88);
a Φ half gear (98) and a Φ-stage bracket (99) each one attached to the prostate module (81);
an optical encoder module (89) and encoder connector (96).

8. The robotic device of claim 7, wherein said prostate module (81) comprises a Ψ-stage (105) and a ZZ-stage (118), said ZZ-stage being adapted to move an endorectal probe (103) along the ZZ-axis, wherein ZZ-axis is the axis of said prostate module, said endorectal probe includes a prostate transducer (104), and said Ψ-stage being adapted to angulate said endorectal probe (103) about said ZZ-axis.

9. The prostate module of claim 8, wherein said Ψ-stage comprises:
a fifth piezoelectric motor (106);
a holder (107) supporting the piezoelectric motor;
a Ψ-pinion gear (108), a Ψ-spur gear (109),
a Ψ-second pinion gear (110) and a Ψ-output gear (111);
an optical encoder (1 14) and encoder connector (130).

10. The robotic module of claim 8, wherein said ZZ-stage comprises:
a sixth piezoelectric motor (119);
a holder (120) supporting the piezoelectric motor;
a ZZ-jack screw (121) attaches to the ZZ-motor (122);
an optical encoder module (123), and encoder connector (131).

11. The robotic device as recited in claim 6, being placeable on MRI table and guidable by MRI, wherein in use said container is filled with degassed water so that ultrasonic energy is acoustically coupled to the target, being adapted for accessing brain, breast, abdominal, bone, and fibroid targets.

12. The robotic device as recited in claim 8, being placeable on MRI table and guidable by MRI, wherein in use said endorectal probe is covered by an ultrasound transparent membrane and filled with degassed water so that ultrasonic energy is acoustically coupled to the target, being adapted for accessing prostate targets.

13. The robotic device of claim 1, guidable by magnetic resonance imaging comprising:
a magnetic resonance (MR) imaging means for imaging brain, bone, prostate, breast, fibroid, and
abdominal sites and for creating temperature sensitive images of ablated sites during surgery;
